**Europäisches Patentamt**

(19) **European Patent Office**

**Office européen des brevets**

(11) Numéro de publication: **0 162 796**
**B1**

(12) **FASCICULE DE BREVET EUROPEEN**

(45) Date de publication du fascicule du brevet:
09.03.88

(21) Numéro de dépôt: **85450008.9**

(22) Date de dépôt: **05.04.85**

(51) Int. Cl.⁴: **B 01 J 8/22,** C 02 F 3/12,
C 02 F 3/28, C 12 M 1/40

(54) Procédé et installations pour fluidiser, expanser ou agiter un lit.

(30) Priorité: **10.04.84 FR 8406011**

(43) Date de publication de la demande:
**27.11.85 Bulletin 85/48**

(45) Mention de la délivrance du brevet:
**09.03.88 Bulletin 88/10·**

(84) Etats contractants désignés:
**AT BE CH DE FR GB IT LI LU NL SE**

(56) Documents cités:
**EP - A - 0 090 450**
**FR - A - 1 444 959**
**FR - A - 2 381 824**

(73) Titulaire: **Coulom, Thierry, 108 bis avenue des Minimes,
F-31200 Toulouse (FR)**
Titulaire: **Lussagnet, Pierre, 5 rue Romain Roland,
F-34200 Sete (FR)**

(72) Inventeur: **Coulom, Thierry, 108 bis avenue des Minimes,
F-31200 Toulouse (FR)**
Inventeur: **Lussagnet, Pierre, 5 rue Romain Roland,
F-34200 Sete (FR)**

(74) Mandataire: **Ravina, Bernard, Cabinet Bernard
RAVINA 24, boulevard Riquet, F-31000 Toulouse (FR)**

ACTORUM AG

**Description**

La présente invention concerne un procédé pour fluidiser des particules composant un lit ascendant dans un milieu liquide, celles-ci présentant un moyen pour favoriser l'accrochage et le développement de micro-organismes dans des processus biologiques. Elle concerne également des installations de mise en oeuvre du procédé.

Le traitement biologique en continu ou non des liquides en vue d'y effectuer certaines transformations est très répandu dans les industries agro-alimentaires, par exemple, bière, vins, tous aliments sous forme liquide, dans les industries pharmaceutiques, dans le traitement d'eau polluée à la suite de certaines opérations de production industrielle ou dans le traitement de l'eau usée des zones urbaines.

Dans ce genre de traitement des micro-organismes peuvent être ajoutés dans le milieu pour effectuer certaines transformations spéicifiques comme c'est le cas généralement dans les industries agro-alimentaires, les industries pharmaceutiques et le traitement d'eau polluée à la suite de certaines opérations de production industrielle.

Dans l'épuration biologique des eaux usées des zones urbaines, par le procédé des boues activées, on provoque la croissance et on maintient la biomasse libre dans laquelle vivent des micro-organismes qui dégradent les matières organiques contenues dans les eaux usées et les transforment en les assimilant à cette biomasse qui est par la suite soit décantée, soit écumée à la surface où on la fait flotter.

Cependant le procédé des boues activées ne permet pas d'atteindre des concentrations en micro-organismes très élevées. Or, il est connu que par l'introduction dans une biomasse libre ou dans un milieu liquide à traiter biologiquement, de particules servant de support d'accrochage, la concentration en micro-organisme augmente et par conséquent le rendement des installations augmente aussi.

Le support d'accrochage composé de particules formant un lit dans le milieu liquide est fluidisé à l'aide d'un courant de ce dernier qui s'oppose à la tendance naturelle de ces particules, soit de flotter, soit d'être précipitées selon leur densité relative par rapport à ce liquide.

Si ces particules sont plus denses que le liquide dans lequel elles sont ajoutées, on les fluidise en les poussant vers le haut à l'aide d'un courant ascendant, on obtient ainsi un lit aux particules ascendantes ou simplement un lit ascendant dont la base se trouve vers le bas.

Dans le cas inverse on obtient un lit descendant dont la base se trouve vers le haut.

Le courant de liquide mobilisant les particules du lit peut être engendré à l'aide d'une pompe qui injecte du liquide vers la base du lit en l'aspirant du côté opposé à cette base dans un circuit fermé.

La tendance est d'utiliser les lits descendants dont les particules sont moins denses que le liquide dans lequel elles sont ajoutées (voir GB 2014551). La raison pour laquelle ces lits descendant sont préférés est d'éviter l'entraînement des particules hors du lit dans le cas d'un lit ascendant soit par le dégagement gazeux lors d'une réaction anaérobie soit par les bulles d'air injectées pour aérer les dits lits lors d'une réaction aérobie. Or d'après nos expériences, on a constaté que l'entraînement des particules hors du lit se reproduit dans les deux cas: lit ascendant ou lit descendant.

En effet, le dit entraînement hors du lit est essentiellement dû au changement de la taille et de la densité relative des dites particules à cause du développement des micro-organismes «biomasse» autour d'elles. Le changement de la densité relative dû à l'accrochage des bulles gazeuses autour de ces particules est relativement négligeable, vu la taille des particules et la proportion de gaz qui peut s'y accrocher soit dans le cas où on y injecte l'air (ou de l'oxygène) pour l'aération soit dans le cas où un dégagement gazeux s'y produit.

Dans le cas d'un lit descendant, si le courant de liquide engendré pour s'opposer au flottement du lit n'est pas suffisant, une partie de celui-ci sera exposé à l'ambiance, ce qui provoque un colmatage à ce niveau là, de même dans le cas d'arrêt du courant liquide, pour une raison quelconque, les particules constituant le dit flotteront, ce qui nécessite de prévoir un dispositif pour empêcher le débordement des dites particules.

Pour faire fonctionner un lit soit descendant soit ascendant en continu, les fonctionnements suivants doivent être assurés:

1. Séparation du liquide traité des particules entraînées.

2. Nettoyage en continu d'une portion des dites particules.

3. Reinjection des particules entraînées.

Dans le cas d'un lit descendant, le premier fonctionnement est assuré par la coopération d'un dispositif faisant passer le liquide seulement vers la sortie avec le courant interne. Ce genre de dispositif est classiquement connu et il est souvent en forme de chicane.

Dans le cas d'un lit descendant, le deuxième fonctionnement peut être assuré à l'aide d'un agitateur qui décroche les micro-organismes des particules.

A ce niveau là, il faut signaler que pour le bon fonctionnement d'un lit fluidisé en mode aérobie, l'épaisseur de la couche de micro-organismes déposée sur le particule doit être controlée, une couche épaisse entraîne des réactions anaérobies à l'intérieur d'elle-même. Ce contrôle est difficilement réalisable pour un lit descendant.

Généralement quel que soit le mode utilisé; aérobie ou anaérobie, la dite épaisseur doit être contrôlée de manière à ce que la couche soit active sur toute son épaisseur.

Le troisième fonctionnement peut être assuré par ces deux genres de lit, à efficacité égale, pour une vitesse égale du courant du liquide.

Dans le cas d'un lit descendant fonctionnant en mode aérobie, l'oxygénation est faite préalablement dans une tour d'oxygénation (voir GB 2614555).

En effet, cette oxygénation est souvent insuffisante pour effectuer une réaction aérobie notamment pour des colonnes de grande hauteur. Si on injecte directement l'air dans la colonne qui contient le lit, cette injection s'oppose au courant du liquide est l'affaiblit. De même dans le cas de dégagement gazeux, dans un lit descendant fonctionnant en mode anaérobie, ce dégagement s'oppose au courant du liquide.

Les particules utilisées pour constituer ces lits sont des particules d'une densité relative, par rapport au liquide qui les contient, soit supérieure, soit inférieure à l'unité.

Notons que ces liquides sont souvent des liquides aqueux d'une densité voisine de 1g/cm$^3$. A l'heure actuelle les particules utilisées ayant une densité inférieure à, mais voisine de 1g/cm$^3$ sont essentiellement constituées de matière synthétique, beaucoup plus chère que celles ayant une densité supérieure à, mais voisine de 1g/cm$^3$ et qui sont souvent constituées de cette matière naturelle par exemple l'argile expansée. Notons que des particules minérales d'une densité inférieure à 1g/cm$^3$ et d'un prix modéré posent le problème d'être mouillable et de changer leur densité en permanence, ce qui rend leur emploi difficile.

On connait également le brevet EP 0090450 qui décrit une installation de traitement d'eaux usées à l'aide d'un lit fluidisé ascendant. Ce lit est fluidisé à l'aide d'un courant ascendant résultant du flux d'alimentation d'une part et du flux de recirculation d'autre part. Un injecteur de gaz est, pour des raisons d'aération, placé à la base du dit lit.

Le flux de recirculation peut être assuré soit par une pompe soit par l'énergie fournie par le flux d'alimentation lui-même.

Pour toutes ces raisons la présente invention visant à pallier les inconvénients ci-dessus énoncés, en ce qui concerne les lits descendants et à simplifier la fluidisation, en ce qui concerne les lits ascendants, propose un procédé et des installations pour fluidiser un lit composé de particules non-flottantes dans un milieu liquide, la fluidisation des dites particules étant réalisée sous l'effet d'un courant du liquide ascendant pour réaliser un lit ascendant.

A cet effet et selon l'invention, le procédé se caracterise par:

– une première injection gaseuse effectuée à la base d'un contenant autre celui qui contient le lit, ayant comme rôle de créer un courant de liquide ascendant, ce courant étant injecté à la base de lit à fluidiser contenu dans un autre contenant, en s'ajoutant au flux qui traverse des contenants reliés entre eux dans le sens entrée-sortie;

– une éventuelle deuxième injection gazeuse effectuée à la base du lit ayant comme rôle d'agiter la dite base et fournir l'oxygène nécessaire dans le cas d'une traitement aérobie, cette éventuelle deuxième injection créant, elle aussi un courant s'ajoutant à celui créé par la première injection gazeuse et à celui de l'alimentation.

Ces contenants sont placés au même niveau ou à des niveaux différents, dans le deuxième cas le contenant possédant un lit étant placés, à un niveau inférieur à celui de l'autre.

Suivant une autre disposition de l'invention, les installations pour traiter biologiquement des liquides se caractérise essentiellement par au moins deux contenants:

Un contenant qui contient un lit à fluidiser dont la base est au niveau bas de ce contenant, ce dernier étant muni à sa base d'un eventuelle injection gazeuse.

Un contenant dans lequel un courant de liquide, s'associant avec le courant qui traverse les contenants dans le sens entrée – sortie, est engendré grâce à une injection gazeuse.

Les liaisons entre ces contenants étant:

Une liaison entre le niveau haut du deuxième contenant et le niveau bas du premièr contenant,

une liaison entre le niveau bas du deuxième contenant et le niveau haut du premièr contenant.

La présente invention sera mieux comprise à la lecture de la description détaillée ci-après du procédé et de quelques exemples de réalisation des installations mettant en oeuvre le procédé donnés à titre d'exemple explicatifs, jointe à des dessins dans lesquels:

La fig. 1 est un schéma des installations, il y a deux tubes coaxiaux, une tube central occupe la plupart de la place intérieure, dans ce tubé un lit ascendant est placé dans la partie inférieure, une injection gazeuse est effectuée pour créer un courant de liquide ascendant dans la partie supérieure, l'espace entre ces deux tubes sert à les relier.

On peut considérer le tube central comme deux contenants superposés, l'inférieur contient le lit ascendant et le supérieur est utilisé essentiellement pour créer le courant de liquide par l'injection gazeuse.

La fig. 2 est un schéma des installations semblables à celles de la fig. 1 sauf que le tube central occupe une place réduite, le lit ascendant et l'injection gazeuse sont installés dans le contenant formé dans l'espace entre les deux tubes.

La fig. 3 est un schéma des installations à deux contenants reliés par des tuyaux.

La fig. 4 est un schéma des installations semblables à celles de la fig. 1 et dans lesquelles le contenant (2) est d'un diamètre réduit pour accentuer l'effet de création d'un courant ascendant et la sortie est munie d'une chicane (3).

Le procédé selon l'invention consiste à créer un courant de liquide par une injection gazeuse dans un premier contenant qui contient ce liquide. Ce courant, en s'associant avec le courant qui traverse les contenants dans le sens entrée-sortie et avec un courant qui resulte de l'injection gazeuse utilisée pour l'aération et/ou l'agitation, sert à fluidiser, un lit ascendant composé par des particules d'une forme quelconque et renfermée dans un autre contenant, ce dernier étant relié au premier contenant.

Le courant crée par l'injection gazeuse dans un contenant parcourt la hauteur de ce dernier, passe par la liaison entre lui et le contenant portant le lit pour être injecté dans la base de ce lit.

Il passe de nouveau par la liaison qui relie le contenant du lit vers un niveau bas du premier contenant.

Dans le cas d'un traitement aérobie ou anaérobie, une injection d'air, d'oxygène ou d'un gaz inerte est effectuée, selon le cas, à la base du lit. Le courant qui en résulte a toujours le même sens que le premier.

Un ensemble composé de deux contenants comme on vient de le décrire est une unité qui peut fonctionner de façon autonome. Plusieurs unités peuvent être reliées ensemble soit en parallèle, soit en série, soit en mélange de ces deux systèmes.

Un ou plusieurs contenants dans lesquels un courant de liquide est engendré peut ou peuvent être reliés à un ou plusieurs contenants dans lesquels sont placés un (des) lit(s) à fluidiser en respectant la règle de liaison entre les contenants: d'une part entre un niveau haut d'un contenant qui ne renferme pas de lit et le niveau bas d'un contenant qui renferme un lit et d'autre part entre le niveau bas d'un contenant qui ne renferme pas de lit et le niveau haut d'un contenant qui renferme un lit.

Les particules s'échappant d'un lit sont portées par le courant résultant d'une part de flux qui traverse les contenants dans le sens entrée-sortie et d'autre part du flux resultant d'une ou des injections gazeuses pour être injectées de nouveau dans la base d'un ou des lits.

L'injection d'air, d'oxygène ou de gaz inerte à la base du lit est effectuée pour agiter la dite base en vue d'aérer et/ou nettoyer les particules à ce niveau là. Donc on aura un lit dont la base est agitée et constituée de particules propres et dont le sommet est beaucoup moins agité et constitué de particules couvertes de la couche de micro-organismes la plus épaisse.

Les injections gazeuses, soit à la base d'un lit soit dans un contenant qui ne renferme pas de lit, s'entraident et se complètent. Une de ces injections peut être diminuée tandis que l'autre est augmentée pour régler l'agitation du lit ou bien son degré de fluidisation.

Dans un traitement anaérobie, ces deux types d'injection gazeuses, l'une à la base d'un lit et l'autre dans un contenant qui ne renferme pas de lit viennent s'ajouter au dégagement gazeux dû à la réaction biologique elle-même. Il est bien entendu que les injections gazeuses, dans ce cas, seront effectuées avec des gaz inertes vis à vis de micro organismes.

On sait que la construction de contenants en hauteur est couteuse pour cela plusieurs contenants qui contiennent des lits peuvent être reliés en série ou en parallèle, un de ces contenants serait relié au contenant dans lequel un courant de liquide est engendré à la suite d'une injection gazeuse. Dans ce cas, les contenants possédant chacun un lit présentent un seul contenant divisé en plusieurs, mais le coût de construction est moins élevé.

Une entrée de liquide à traiter est branchée à au moins un de ces contenants.

C'est ainsi qu'on obtient un ensemble de traitement biologique de liquides composé d'au moins un contenant dans lequel une injection gazeuse est effectuée pour créer un courant ascendant de liquide, d'au moins un contenant lié au précédent dans lequel un lit ascendant à fluidiser est placé et dans la base de laquelle une injection gazeuse d'aération et/ou d'agitation est effectuée, une entrée de liquide à traiter et une sortie de liquide est traité.

L'entrée de liquide à traiter est branchée, de préférence de façon à assurer que ce liquide fasse le parcours du lit avant d'atteindre la sortie.

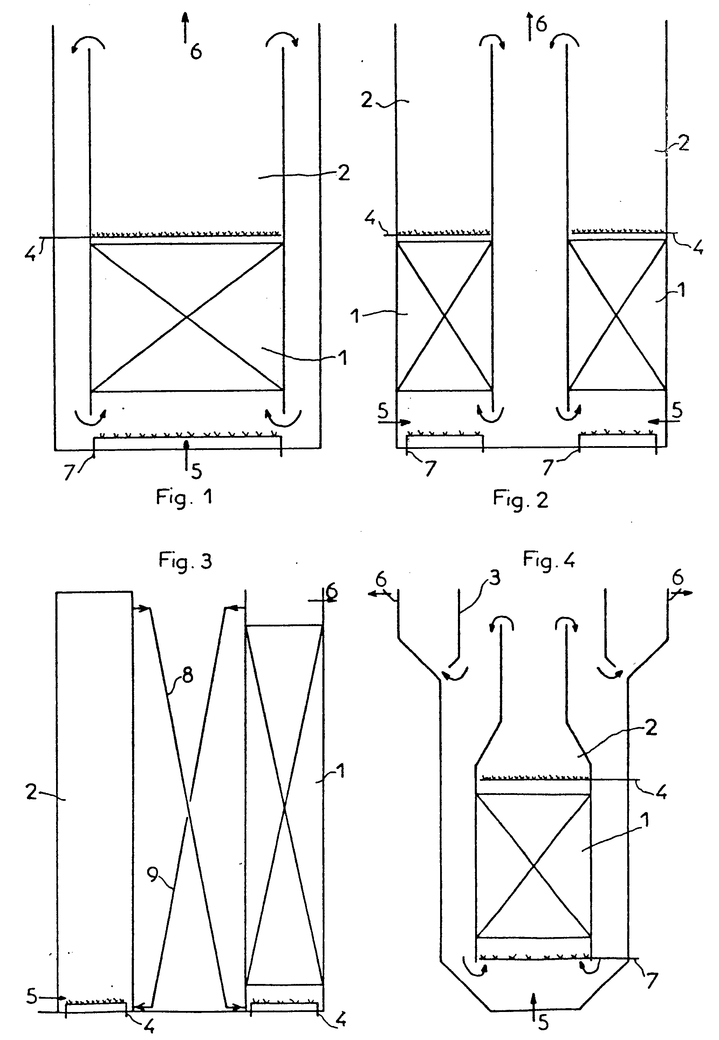

Ce branchement peut être effectué sur un contenant qui renferme un lit et au niveau de base de celui-ci comme dans les fig. 1 et 2 ou dans un contenant dans lequel le courant de liquide est essentiellement provoqué comme dans la fig. 3.

Les contenants peuvent être placés au même niveau comme dans la fig. 3, ou peuvent être placés à des niveaux différents comme montrent les fig. 1, 2 et 4:

Les contenants qui renferment des lits sont au niveau inférieur et ceux qui n'en renferment pas sont au niveau supérieur comme dans les fig. 1, 2 et 4.

L'injection gazeuse effectuée dans les contenants qui ne renferment pas de lit est de préférence à un niveau bas de ceux-ci de façon à atteindre une vitesse de flux capable de fluidiser le lit en s'associant avec le courant de liquide qui traverse les contenants, avec un débit minimal de gaz.

Dans le cas de traitement anaérobie de liquides, l'injection gazeuse peut être effectuée en circuit fermé avec un gaz inactif par rapport aux micro-organismes existant utilisés dans le milieu.

Sur la fig. 1 est présentée une forme de réalisation de ces installations.

Elles sont constituées de deux tubes coaxiaux le tube central qui a un diamètre légèrement inférieur que celui de tube extérieur représente deux contenants 1 et 2 superposés ouverts à leurs extrémités et se communiquant entre eux.

Le contenant inférieur 1 contient le lit à fluidiser, le contenant supérieur contient le flux de liquide crée à l'aide d'une injection gazeuse par le diffuseur 4.

L'espace entre les deux tubes coaxiaux sert à relier le niveau supérieur du contenant 2 au niveau inférieur du contenant 1.

Une entrée 5 du liquide à traiter est branchée au niveau inférieur du contenant 1.

Une sortie 6 du liquide traité est branchée au niveau supérieur du contenant 2.

Un diffuseur 7 est prévu pour aérer les micro-organismes accrochés au lit, dans le cas d'un traitement aérobie et pour agiter la base du lit, dans le cas de traitement aérobie ou anaérobie.

La fig. 2 est une reprise des installations présentées sur la fig. 1:

L'espace entre les deux tubes présente dans ce cas les deux contenants 1 et 2.

Les positions relatives de l'injection gazeuse créatrice du flux, de l'injection gazeuse de l'aération et/ou de l'agitation de l'entrée du liquide à traiter et de la sortie du liquide traité sont conservées.

La fig. 3 représente une autre réalisation de ces installations.

Un contenant 1 qui renferme le lit à fluidiser et un contenant 2 qui contient le courant ascendant du liquide crée par l'injection gazeuse sont reliés par:
– Un tuyau 8 reliant le niveau haut du contenant 2 au niveau bas du contenant 1,
– un tuyau 9 reliant le niveau haut du contenant 1 au niveau bas du contenant 2.

Une entrée 5 de liquide à traiter est branchée au niveau bas du contenant 2 et une sortie 6 du liquide traité est branchée au niveau haut du contenant 1.

La diffusion gazeuse pour créer le flux de liquide est effectuée à travers un diffuseur 4.

Un autre diffuseur 7 est prévu pour aérer et/ou agiter le lit sur lequel les micro-organismes sont accrochés.

La fig. 4 représente les mêmes installations que la fig. 1 dans lesquelles le contenant 2 a un diamètre réduit pour accentuer la création du courant du liquide et la sortie 16 est pourvue d'une chicane 3.

Dans tous les exemples que l'on vient de décrire, un flux de liquide à traiter est crée essentiellement dans un contenant 2 à l'aide d'une injection gazeuse effectuée à un niveau bas de celles-ci, ce flux s'associe avec celui qui traverse le contenant dans le sens entrée-sortie, dès que le flux total atteint le niveau haut de ce contenant, il est injecté à la base d'un lit composé des particules d'une forme quelconque dans un autre contenant 1. Le flux passe à travers ce lit pour être injecté partiellement à un niveau bas du premier contenant 1.

Le débit de liquide à l'entrée 5 est pratiquement égal au débit de celui-ci à la sortie 6.

Dans le cas de traitement anaérobie, l'injection gazeuse pour créer le flux dans le contenant 2 peut être effectuée dans un circuit fermé avec un gaz inactif par rapport aux micro-organismes utilisés.

Dans ce dernier cas, une séparation chimique ou physique du gaz injecté d'éventuels gaz dégagé du aux processus biologique peut être envisagée. Cette étape de séparation doit être associé au dit circuit fermé.

Toutes modifications classiques peuvent être apportées aux dites installations a savoir, dans le cas de traitement biologique des eaux usées de zones urbaines, et des eaux polluées à la suite de certaines opérations de productions industrielle, pour enrichir le milieu, une réinjection des flocons de biomasse s'échappant par la sortie, peut être envisagée dans un des deux contenants.

D'autres modifications propres à chaque application qui sont connues par l'homme de l'art peuvent être encore envisagées. Une combinaison quelconque d'au moins un contenant de type du contenant 1 et au moins un contenant du type du contenant 2 peut être réalisée en respectant la règle de liaison entre ces contenants.

## Revendications

1. Procédé pour fluidiser un lit composé des particules non-flottantes dans un milieu liquide, ces dernières étant ajoutées en vue de favoriser l'accrochage et le développement de micro-organismes dans des processus biologiques, la fluidisation des dites particules étant effectuée sous l'effet du courant ascendant, procédé étant caractérisé par:
– une première injection gazeuse effectuée à la base d'un contenant autre que celui qui contient le lit, ayant comme rôle de créer un courant de liquide ascendant, ce courant étant injecté à la base du lit à fluidiser, en s'ajoutant au flux qui traverse des contenants reliés entre eux dans le sens entrée-sortie
– une éventuelle deuxième injection gazeuse effectuée à la base du lit ayant comme rôle d'agiter la dite base et fournir l'oxygène nécessaire dans le cas d'une traitement aérobie, cette éventuelle deuxième injection créant, elle-aussi, un courant s'ajoutant à celui créé par la première injection gazeuse et à celui de l'alimentation.

2. Procédé selon la revendication 1, caractérisé en ce que les dits contenants sont placés au même niveau ou à des niveaux différents dans le deuxième cas, le contenant possédant le lit étant placé à un niveau inférieur à celui de l'autre.

3. Installation pour traiter biologiquement des liquides avec des lits fluidisés, selon le procédé défini dans les revendications 1 et 2, caractérisé par au moins deux contenants:
– Un contenant (1) qui contient le lit dont la base est au niveau bas de ce contenant, ce dernier étant muni à sa base d'une éventuelle injection gazeuse;
– un contenant (2) dans lequel un courant de liquide est engendré grace à une injection gazeuse.

Les liaisons entre ces contenants étant:
– Une liaison entre le niveau haut du contenant (2) et le niveau bas du contenant (1);
– une liaison entre le niveau bas de la contenant (2) et le niveau haut du contenant (1).

4. Installation selon la revendication 3, caractérisé en ce que le contenant (1) et le contenant (2) sont soit au même niveau (fig. 3) soit à deux niveaux différents (fig. 1, 2 et 4), le contenant (1) étant au niveau inférieur à celui du contenant (2).

## Claims

1. Method for fluidizing a bed formed of non floating particles in a liquid medium, these latter being added for promoting the attachment and development of microorganisms in biological processes, the fluidization of said particles being effected under the effect of the rising stream, which method is characterized by:
– a first gas injection made at the base of a container other than the one which contains the bed, whose purpose is to create a rising liquid stream, this stream being injected at the base of the bed to be fluidized, and adding itself to the flow which passes through containers connected together in the input-output direction,

– possibly a second gas injection made at the base of the bed for stirring said bed and supplying the oxygen required in the case of an aerobic treatment, this possible second injection also creating a stream which is added to that created by the first gas injection and to that of the supply.

2. Method according to claim 1, characterized in that said containers are placed at the same level or at different levels in the second case, the container having the bed being placed at a lower level than that of the other.

3. Installation for biologically treating liquids with fluidized beds, in accordance with the method defined in claims 1 and 2, characterized by at least two containers:

– one container (1) which contains the bed whose base is at the lower level of this container, this latter being provided at its base with a possible gas injection,

– a container (2) in which a liquid stream is generated through gas injection, the connections between these containers being:

– a connection between the top level of the container (2) and the low level of the container (1)

– a connection between the low level of the container (2) and the top level of container (1).

4. Installation according to claim 3, characterized in that the container (1) and the container (2) are either at the same level (3) or at two different levels (fig. 1, 2 and 4), container (1) being at a level lower than that of container (2).

**Patentansprüche**

1. Verfahren zur Aufwirbelung eines Bettes aus nicht schwimmenden Teilchen in einem flüssigen Milieu, in dem die Teilchen hinzugefügt werden, um das Haften und Entwickeln von Mikroorganismen in biologischen Prozessen zu fördern, und in dem die Fluidisierung der besagten Teilchen unter Einwirkung eines aufsteigenden Stromes bewirkt wird, Verfahren gekennzeichnet durch:

– eine erste Gaseinspritzung an der Basis eines anderen Behälters als dem, der das Wirbelbett enthält, mit der Aufgabe, einen aufsteigenden Flüssigkeitsstrom zu erzeugen, wobei dieser Strom an der Basis des zu fluidisierenden Bettes eingespritzt wird und sich zum Strom addiert, der die miteinander verbundenen Behälter in Richtung Eingang-Ausgang durchfliesst,

– eine eventuelle zweite Gaseinspritzung an der Basis des Bettes mit der Aufgabe, die besagte Basis zu durchmischen und den für eine Aerobiebehandlung erforderlichen Sauerstoff zu liefern, wobei diese eventuelle zweite Einspritzung ebenfalls einen Strom erzeugt, der sich zu dem mit der ersten Gaseinspritzung erzeugten und dem der Versorgung addiert.

2. Verfahren gemäss Anspruch 1, dadurch gekennzeichnet, dass die besagten Behälter in gleicher Ebene oder in verschiedenen Ebenen angeordnet sind, wobei im zweiten Fall der Behälter des Wirbelbettes in einem niedrigeren Niveau als der andere angeordnet ist.

3. Verfahren für die biologische Aufbereitung von Flüssigkeiten mit Wirbelbetten nach dem in den Ansprüchen 1 und 2 bestimmten Verfahren, durch mindestens zwei Behälter gekennzeichnet:

– Einen Behälter (1), der das Bett enthält, dessen Basis sich in der unteren Ebene dieses Behälters befindet, und der an seiner Basis mit einer eventuellen Gaseinspritzung versehen ist,

– einen Behälter (2), in dem durch Gaseinspritzung ein Flüssigkeitsstrom erzeugt wird, wobei die Verbindungen zwischen diesen beiden Behältern wie folgt ausgeführt sind:

– Eine Verbindung zwischen der oberen Ebene des Behälters (2) und der unteren Ebene des Behälters (1),

– eine Verbindung zwischen der unteren Ebene des Behälters (2) und der oberen Ebene des Behälters (1).

4. Installation gemäss Anspruch 3, dadurch gekennzeichnet, dass der Behälter (1) und der Behälter (2) auf der gleichen Ebene (Fig. 3) oder auf zwei verschiedenen Ebenen (Fig. 1, 2 und 4) sind, wobei der Behälter (1) auf einer niedrigeren Ebene als der Behälter (2) angeordnet ist.

0162796

Fig. 1

Fig. 2

Fig. 3

Fig. 4